(19)

**Europäisches Patentamt**
**European Patent Office**
**Office européen des brevets**

(11)  **EP 3 912 677 A1**

(12)  # EUROPEAN PATENT APPLICATION

(43) Date of publication:
**24.11.2021 Bulletin 2021/47**

(21) Application number: **20382416.4**

(22) Date of filing: **18.05.2020**

(51) Int Cl.:
*A61N 1/36* (2006.01)          *A61B 5/0488* (2006.01)
*A61B 5/04* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Consejo Superior de Investigaciones Científicas
(CSIC)**
**28006 Madrid (ES)**
• **Imperial College Innovations Limited
London Middlesex SW7 2AZ (GB)**
• **Fundación para la Investigación Biomédica del
Hospital Gregorio Marañón (FIBHGM)**
**28007 Madrid (ES)**

(72) Inventors:
• **PONS, Jose Luis**
28002 Madrid (ES)
• **OLIVEIRA BARROSO, Filipe André**
28002 Madrid (ES)
• **PASCUAL VALDUNCIEL, Alejandro**
28002 Madrid (ES)
• **FARINA, Dario**
London, Middlesex SW7 2AZ (GB)
• **GRANDAS, Francisco**
28007 Madrid (ES)

(74) Representative: **Pons**
**Glorieta Rubén Darío 4**
**28010 Madrid (ES)**

(54)  **CONTROL METHOD FOR A NEUROPROSTHETIC DEVICE FOR THE REDUCTION OF
PATHOLOGICAL TREMORS**

(57)      The invention relates to a control method for a neuroprosthetic device, allowing to monitor and reduce pathological tremors in users via the stimulation of the peripheral muscles and modulation of the afferent pathways.

EP 3 912 677 A1

**Description**

The field of the invention falls within the scope of computer implement methods in medical science, more specifically in electrotherapy methods. The invention relates to a control method for a neuroprosthetic device, allowing to monitor and reduce pathological tremors in users via the stimulation of the peripheral muscles and modulation of the afferent pathways.

**BACKGROUND ART**

Pathological tremor can be defined as a strong involuntary oscillatory movement of a body part that is produced by alternating contractions of reciprocally innervated muscles. Tremor is the most common movement disorder and its incidence and prevalence are increasing with the aging of the world population, affecting 15% of people aged between 50 and 89 years. The most common are tremors caused by the two neurodegenerative diseases: Parkinson's disease (PD) and Essential tremor (ET). Although the disorder is not life-threatening, more than 65% of population suffering from upper limb tremor reports serious difficulties in performing the activities of daily living (ADL), greatly decreasing their independence and quality of life.

Pathological tremors - from now onwards referred simply as tremors - underlying mechanisms are not fully understood mostly due to a lack of understanding on their etiology. The main tremor management approach is medication. In this regard, first line treatments with established efficacy are propranolol and primidone, although average reduction of tremor using these medications is approximately 50%, and one in three patients ends up discontinuing treatment (Shanker V., BMJ. August 2019:14485). For these cases, there are alternative second-line therapies, such as deep brain stimulation (DBS) or high-intensity focused ultrasound (Ferreira et al., Mov Disord. 2019; 34(7): 950-958). DBS requires a highly complex and invasive neurosurgical intervention, while HIFU creates an ablative lesion in the central nervous system (CNS), with the impossibility of reversing or regulating its effect. Additionally, both interventions are still highly expensive and cannot be performed in all patients.

Recent developments have introduced techniques alternative treatments for pathological tremors. The patent document US20060212089 describes a device for the desynchronization of brain areas. The patent document US2006217781 describes a series of methods for the treatment of brain disorders, like movement disorders, through modulation of brain networks and structures. The patent document US2010087698 describes repetitive transcranial magnetic stimulation over a patient's scalp to treat muscle movement disorder. The document US2011112394A1 describes methods and systems for non-invasive deep brain or superficial neuromodulation through the use of ultrasound stimulation. The techniques mentioned so far act directly on the brain and despite good results having been observed, they suffer from side effects associated with brain activity modulation.

For such reasons, other techniques for the treatment of tremors have focused on the stimulation of afferent pathways, i.e., the sensory nerves. The patent document US2011184489 describes a method for the treatment of disorders associated with Parkinson's disease based in the continuous stimulation of the spinal cord. The patent document US2017014625 describes systems, devices, and methods for the treatment of tremors by the stimulation of peripheral nerves.

The document US20140336722 describes a device and method for the treatment of tremors through neurostimulation of the afferent pathways. The document US20140336722 describes the use of an out-of-phase approach to the stimulation of the afferent pathways where the tremors are detected for a period of time, such detection is used to predicted the muscle tremor behavior in the following period of time and the antagonist muscles are stimulated in the periods of non-tremor according to a predicted tremor pattern. Despite avoiding the side effects from direct brain stimulation, peripheral stimulation techniques suffer from low efficacy. In addition, even in the cases where patients do benefit from the treatments, the effects tend to not last for more than a few minutes after stimulation has ended. Such lack of effectiveness of tremor reduction could be explained by the inconsistence of the phase of muscle tremor patterns over time due to the stretch reflex and mechanical resonance of the limb.

As such, in view of the present state of the art, there is a dire need for alternative techniques for tremor treatments which avoid the side effects of the direct brain stimulation and potentiate the effects of tremor reduction of peripheral stimulation techniques to more than a few minutes post stimulation.

**DESCRIPTION OF THE INVENTION**

The present invention discloses a method for controlling a neuroprosthetic device for the treatment of tremors, in particular Essential Tremors (ET) and Parkinson's Disease (PD), by intramuscular stimulation of muscle spindles. This new method allows the monitoring in real time of the tremorgenic bursts at the peripheral agonist muscle and concomitant stimulation of the antagonist muscle. Monitoring and stimulation are made at the peripheral level (muscles) of the nervous system. Preliminary results show that the method of the invention here described allows for the reduction of tremors in patients up to 24h post-treatment without presenting detectable side effects.

Therefore, the present invention solves the problem of short-term effect of tremor reduction in methods based on peripheral stimulation while avoiding the side effect problems associated with direct brain stimulation methods.

A first aspect of the present invention relates to a method for a window-based control of a neuroprosthetic device, from here onwards the "method of the invention", comprising the steps of:

a) obtaining a set of electromyography (EMG) signals from at least one pair of EMG electrodes;
b) filtering the EMG signals obtained by using a low-pass filter;
c) obtaining envelopes of the filtered EMG signals;
d) determining two or more local maxima of the envelopes obtained;
e) calculating period and frequency of tremorgenic bursts of each muscle based on said local maxima;
f) determining an adaptive EMG threshold of tremor for each EMG signal, which is computed as root mean square (RMS) of the EMG signals, obtained in step a), multiplied by a gain between 0.5 and 2;

wherein when frequencies of tremorgenic bursts of one or more muscles are in the range of 3-12 Hz, is determined that tremor is present and a stimulation mode is enabled, which further comprises the steps of:

i) setting a stimulation time-window, which has a duration inversely proportional to the period of tremorgenic burst, such that the shorter the tremorgenic burst, the longer the stimulation window;
ii) obtaining one or more updated EMG signals of one or more muscles from the EMG electrodes;
iii) obtaining an updated RMS value of a measuring time window of each updated EMG signal;
iv) comparing each updated RMS value with the adaptive EMG threshold;
v) determining existence of a tremor in an agonist muscle if the RMS value of an EMG electrode placed on said agonist muscle is above the adaptive EMG threshold,
vi) generating an activation signal on the neuroprosthetic device placed in an antagonist muscle, with respect to the agonist muscle suffering a tremor, repeating steps (ii) to (v) until the duration of the stimulation time-window is finished.

The term "window-based" as used herein refer to algorithms wherein the input data can be considered as an ordered sequence in time, and restriction of computations can be made to portions of the input. Therefore, it is assumed that the input is a stream (i.e., the EMG signals) of data elements and divides the data elements into two categories: active (the second time window of the EMG signals) and expired elements (the first-time window).

The method of the invention can be implemented as an algorithm wherein the term "algorithm" refers to a finite sequence of well-defined computer implemented instructions, used to control the neuroprosthetic device.

The term "neuroprosthetic device" as used herein refers to a device used to replace or improve the function of an impaired nervous system, leading to performance changes on the input or output of a neural system, normally by direct or indirect (through neuromodulation at peripheral level) electrical stimulation. Neuroprosthetics encompass a variety of artificial devices or systems that can be used to enhance the motor, sensory, cognitive, visual, auditory, and communicative deficits that arise from acquired brain injuries. The term "neuromodulation" as used herein refers to the alteration of nerve activity through targeted delivery of a stimulus, such as electrical stimulation or chemical agents, to specific neurological sites in the body.

The term "electromyography (EMG) signal" as used herein refers to electromyography of the skeletal muscles. The term "electromyography" as used herein refers to a recording of the electrical activity (i.e., voltage potential) produced by the skeletal muscles, such activity being recorded by an EMG amplifier, which can be represented by an oscillatory signal as a function of time.

The term "electrode" as used herein refers to an electric conductor through which a voltage potential can be measured. An electrode can also be a collector and/or emitter of an electric current. Electrodes are solid and comprise a conducting metal. Representative conducting metals include noble metals, alloys and particularly stainless steel and tungsten. An electrode can also be a microwire or a thin film, or the term "electrode" can describe a collection of microwires or thin films.

The term "EMG electrode" as used herein refers to an electrode that collects an EMG signal. "EMG electrodes" can be multichannel thin film electrodes, whose channels can collect electric current independently from the other channels.

The step a) "obtaining a set of electromyography (EMG) signals from at least one pair of EMG electrodes" of the method of the invention refers to the collection of the electrical activity of the skeletal muscles through the electrodes therein implanted. In a preferred embodiment of the method of the invention the step a) obtains the EMG signal during a period of 0.5 to 2 seconds. In a further preferred embodiment of the method of the invention the EMG signal is obtained from the agonist skeletal muscle.

The terms "agonist skeletal muscle" or "agonist muscle" and "antagonist skeletal muscle" or "antagonist muscle" as used herein refer to muscles that cause or inhibit a movement. Agonist muscles cause a movement to occur through their own activation while antagonist muscles are the muscles that produce an opposing joint torque to the agonist muscles. Antagonist and agonist muscles often occur in pairs, called "antagonistic pairs". In antagonistic pairs of muscles, the function of agonist or antagonist is not fixed to anyone's muscle as either muscle can act both as agonist and antagonist. The method of the invention determines for each time window which muscle contracts and relaxes, i.e. which muscle functions as the agonist, and which functions as the antagonist.

The term "filtering" as used herein refers to the processing or removing of unwanted components, frequencies, or frequency bands from the "EMG signal", leading to the complete or partial reduction of some feature of the signal. One such type of filtering process is "low-pass filter", a type of filter that passes signals with a frequency lower than a selected cut-off frequency and attenuates signals with frequencies higher than the cut-off frequency. In a preferred embodiment of the method of the invention the low-pass filter is a Butterworth filter. Also called maximally flat magnitude filter, the Butterworth filter is a type of signal processing filter designed to have a frequency response as flat as possible in the passband.

The term "envelope" as used herein refers to a smooth curve outlining the extremes of an oscillatory signal.

The steps b) "filtering the EMG signals obtained by using a low-pass filter", c) "obtaining envelopes of the filtered EMG signals" and d) "determining two or more local maxima of the envelopes obtained" in the method of the invention refer to the necessary steps to transform the raw signal obtained from the muscle activity into a signal from where step e) "calculating period and frequency of tremorgenic bursts of each muscle based on said local maxima" can be performed. In a preferred embodiment the method of the invention further comprises a step of applying a Hilbert transformation to the envelope obtained in step c) for obtaining an improved envelope.

Having determined two or more local maxima of the envelopes obtained, step e) "calculating period and frequency of tremorgenic bursts of each muscle based on said local maxima" of the method of the invention is performed. The term "frequency" as used herein refers to the number of occurrences of a repeating event per unit of time, being the "period" the inverse of the frequency. The term "tremorgenic burst" as used herein refers to a time interval of sudden and intense activity of the muscle.

Step f) of the method of the invention "determining an adaptive EMG threshold of tremor for each EMG signal, which is computed as root mean square (RMS) of the EMG signals obtained in step a) multiplied by a gain between 0.5 and 2", refers to the calculation of the EMG threshold value for distinct time resolved calculations leading to different threshold values, i.e. adaptative EMG threshold, for different times in the execution of the control of the neuroprosthetic device. The term "root mean square (RMS)" as used herein refers to the square root of the arithmetic mean of the squares of the EMG signals. The RMS is also known as the "quadratic mean".

As defined by the method of the invention aspect, if the "frequencies of tremorgenic bursts of one or more muscles are in the range of 3-12 Hz" as determined in step e) of the method of the invention, it "is determined that tremor is present" and the method of the invention enters a stimulation mode which further comprises steps i) to vi). Said steps are performed in order to reduce the level of tremors detected in the patients.

The term "stimulation" as used herein refers to the generation of an activation signal on the neuroprosthetic device, which leads to the delivery of electrical current pulses to the muscles of the patient.

The terms "subject", "patient" and "user" are used interchangeably and as used herein refer to any individual monitoring or employing the present invention, or an element thereof. In the context of the invention, the method of the invention may be applied by a subject to a patient in a clinical context, or may be applied by a user and patient, i.e. a person which self-applies the method of the invention. Subjects can be, for example, researchers gathering data from an individual, an individual who determines the parameters of operation of the present invention or the individual in or on which a high-density multichannel microelectrode array is disposed. Broadly, then, a "subject", "patient" or "user" is one who is employing the present invention for any purpose. As used herein, the terms "subject", "patient" or "user" need not refer exclusively to human beings, but rather the terms encompass all organisms having neural tissue, such as monkeys, dogs, cats, rodents, etc.

The step i) of the method of the invention, "setting a stimulation time-window, which has a duration inversely proportional to the period of tremorgenic burst, such that the shorter the tremorgenic burst, the longer the stimulation time-window", refers to the establishment of a discrete time interval, i.e. time-window, which is inversely dependent or proportional on the time duration of the tremorgenic burst, i.e. the longer (shorter) the tremorgenic burst the shorter (longer) the time period.

Having established said stimulation time-window, step ii) "obtaining one or more updated EMG signals of one or more muscles from the EMG electrodes" is performed. In a preferred embodiment of the method of the invention, the updated EMG signal is obtained during 3 to 15 milliseconds. The updated EMG signals are next process in step iii) of the method of the invention by "obtaining an updated RMS value of a measuring time-window of each updated EMG signal".

Once obtained the RMS value for each updated EMG signal, as stated in step iv) of the stimulation mode of the method of the invention, the updated EMG signals are compared to the adaptative EMG threshold calculated in step f) of the method of the invention. Said comparison will allow to determine if there is a tremor in the muscle from where the updated EMG signal originates, according to step v) of the method of the invention "determining the existence of tremor in an agonist muscle if the RMS value of an EMG electrode placed on said agonist muscle is above the adaptive EMG threshold".

If a determination is made that a tremor exists in the agonist muscle then the next step of the method of the invention, step vi) is "generating an activation signal on the neuroprosthetic device placed in an antagonist muscle, with respect to the agonist muscle suffering a tremor". Said step refers to the initiation of the neuroprosthetic device in response to the detection of tremors, such detection based on the comparison of the RMS value (step iv)) with the adaptative EMG threshold obtained in step f). In a preferred embodiment of the method of the invention, step iv) generates the activation signal during 5 to 20 milliseconds.

The term "activation signal" as used herein refers to an electrical signal of determined intensity and duration which is emitted by the stimulating electrodes in order to activate the muscles where the stimulating electrodes are implanted. In a preferred embodiment of the method of the invention the activation signal is delivered to the antagonist muscles.

In another preferred embodiment the method of the invention further comprises a step of calculating the intensity and duration of the activation signal of the neuroprosthetic device based on the EMG signal and below the motor threshold.

The term "motor threshold" as used herein refers to minimum stimulation intensity that can produce a muscle twitch or contraction. The value of motor threshold is specific for each subject and therefore as to be determined *a priori.*

Finally, the steps i) to vi) of the method of the invention are repeated until the duration of the stimulation time-window, as set in step i), is finished. In a preferred embodiment of the method of the invention steps i) to vi) are performed during 30 seconds before repeating step a).

Another aspect of the invention relates to a computer program, the computer program of the invention, adapted to perform the method of the invention. Yet another aspect of the invention relates to a computer readable storage medium comprising the computer program of the invention.

A neuroprosthetic device, controlled by the method of the invention, for the monitoring and reduction of pathological tremors in a user via the stimulation of the afferent pathways, is one characterized in that it comprises one or more wearable elements on the user, wherein the wearable element comprises two or more EMG electrodes and a programmable electronic device capable of performing the method of the invention.

The term "monitoring" as used herein refers to the observation, and surveillance of the parameters of the pathological tremors of a patient over time.

The term "reduction" as used herein refers to the reduction by a significant amount or the complete stoppage of the pathological tremors. Tremor reduction might be achieved through the acute effect of stimulation or by means of a prolonged neuromodulation effect.

The term "afferent pathways" as used herein refers to the axons of sensory neurons that carry information from the skeletal muscles to the spinal cord. The term "efferent pathways" as used herein refers to the motor neuron axons that carry signals from the spinal cord to the skeletal muscles.

The term "wearable" as used herein refers to smart electronic devices (electronic devices with micro-controllers) that can be incorporated into clothing or worn on the body as implants or accessories. Wearable elements are separated and comprise distinct modules of the programmable electronic device. Wearable elements may further comprise an external device which implements the user interface which allows for the display of data as well as to access to the functionalities of the programmable electronic device of one or more wearable elements.

The term "EMG electrode" as used herein refers to an electrode that collects an EMG signal. "EMG electrodes" can be multichannel thin film electrodes, whose channels can collect electric current independently from the other channels.

The term "programmable electronic device" refers to an electronic device with a microcontroller which allows the method of the invention to be performed. Programmable electronic devices may comprise one or more modules which carry out one or more of the functions of acquiring the EMG signals, process the EMG signals, generating an activation signal, generating a response stimulation, delivering the stimulation signal, flow data control, module control, user interface and user input.

The external device and the wearable elements are connected by a wired connection or wireless connection. The external device may further comprise means for analysis of tremors and generation of diagnostics and reporting, such as algorithms and statistical software, and a memory storage for the measurements made by the neuroprosthetic device.

Several of the terms used in association with the neuroprosthetic device have been previously described in relation to the method of the invention and such definitions are valid in the context of neuroprosthetic device.

The monitoring and reduction of pathological tremors in a patient is carried out by a method characterized by comprising the following stages:

i) characterizing the tremor by means of a neuroprosthetic device which implements the method of the invention, and;

ii) delivering the stimulation signal to the plurality of stimulating electrodes present in the wearable elements of the neuroprosthetic device, wherein the signal is determined by the activation signal obtained by the method of the invention, in order to activate the afferent pathways.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

## DESCRIPTION OF THE DRAWINGS

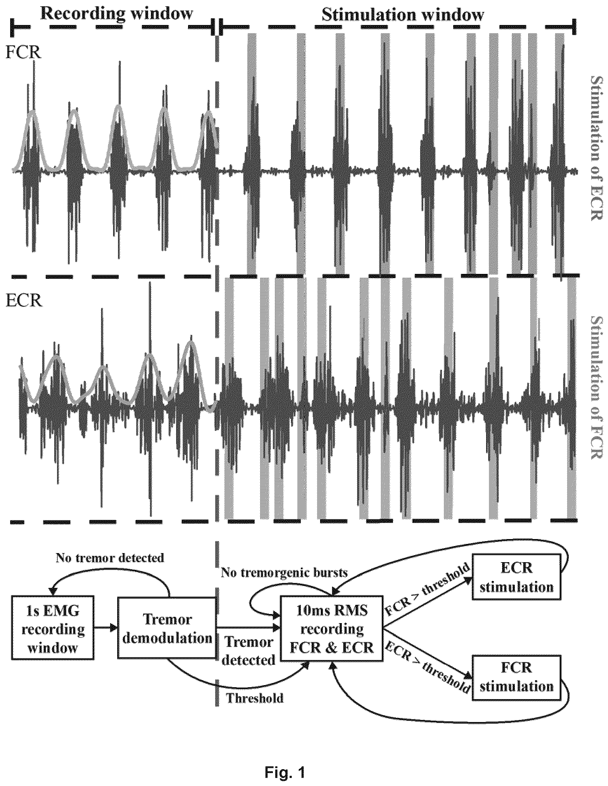

**Fig. 1. Illustration of the selective and adaptive timely stimulation (SATS - the method of the invention) strategy for a pair of tremorgenic muscles controlling the wrist (flexor carpis radialis - FCR, and extensor carpis radialis - ECR).** In the recording window (1s), tremor was detected from the EMG signals. When tremor was present, stimulation was enabled during the subsequent 2s. Although grey areas are drawn over tremorgenic burst activity of the recorded muscle, stimulation pulses were delivered to the antagonist muscle to that where tremorgenic activity was detected in short RMS windows. SATS allows co-contraction of both muscles or individual stimulation of on muscle, depending if both muscles present tremorgenic burst at the same time or not.

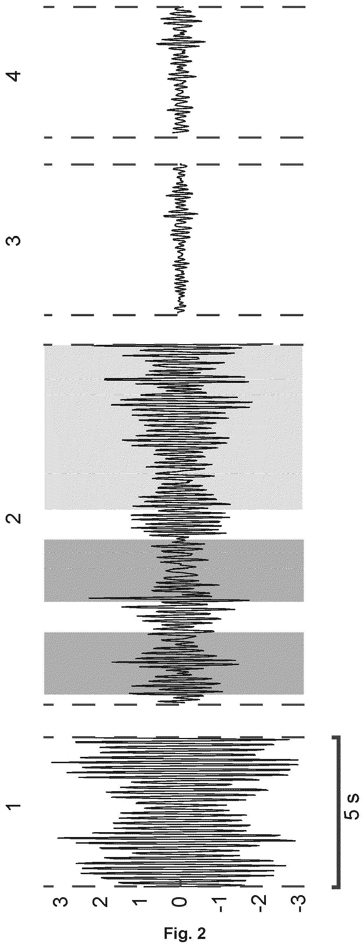

**Fig. 2. Illustration of the effects of electrical stimulation on segments of wrist flexion-extension amplitude (degrees).** Data corresponding to the intramuscular stimulation session with SATS of patient P5. Window 1: Pre-assessment; Window 2: stimulation trial, dark grey areas represent 2s stimulation windows, light grey represents a period when stimulation was turned OFF; Window 3: Post-assessment; Window 4: Assessment 24h after experimentation.

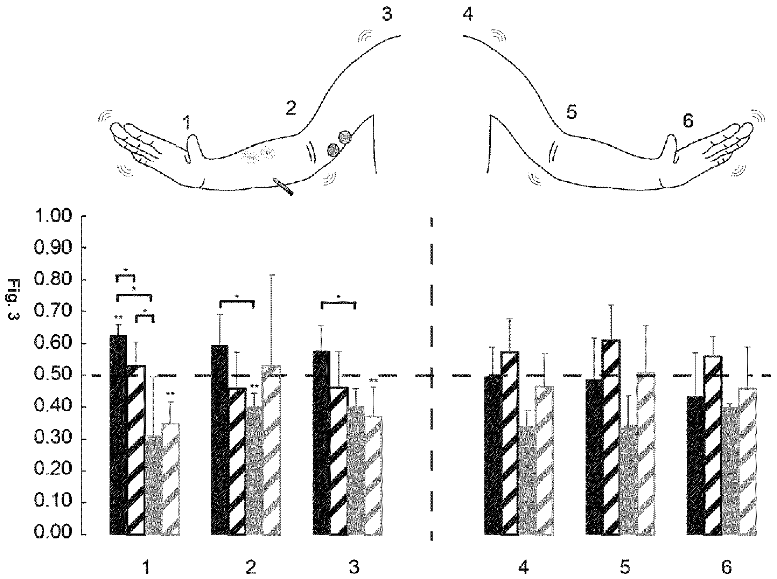

**Fig. 3. Acute tremor reduction scores on kinematics tremor power for 4 ET patients.** Tremor scores between the 30 s OFF period (stimulation turned OFF) and 30 s ON period (stimulation turned ON) for each assessed joint (1-stimulated wrist, 2 ipsilateral elbow, 3 ipsilateral shoulder, 4-contralateral shoulder, 5 contralateral elbow, 6-contralateral wrist). 0.5 represents no change in tremor amplitude, 1 represents 100% tremor reduction and 0 represents 100% tremor aggravation. Black bars: intramuscular SATS stimulation; dashed black bars: surface SATS stimulation; grey bars: intramuscular CON stimulation; dashed grey bars: surface CON stimulation. * Statistical significance between groups ($p < 0.05$);

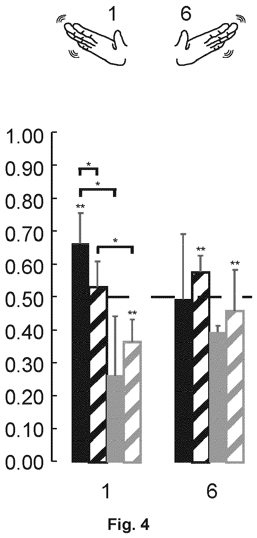

**Fig. 4. Acute tremor reduction scores on kinematics tremor power for 4 ET patients.** Tremor scores between the 30 s OFF period and the 2 s stimulation windows segmented from the ON periods (1-stimulated wrist, 6-contralateral wrist). 0.5 represents no change in tremor amplitude, 1 represents 100% tremor reduction and 0 represents 100% tremor aggravation. Black bars: intramuscular SATS stimulation; dashed black bars: surface SATS stimulation; grey bars: intramuscular CON stimulation; dashed grey bars: surface CON stimulation. * Statistical significance between groups (unpaired T-test, $p < 0.05$); ** Statistical significance from baseline tremor (one-sample T-test, $p < 0.05$).

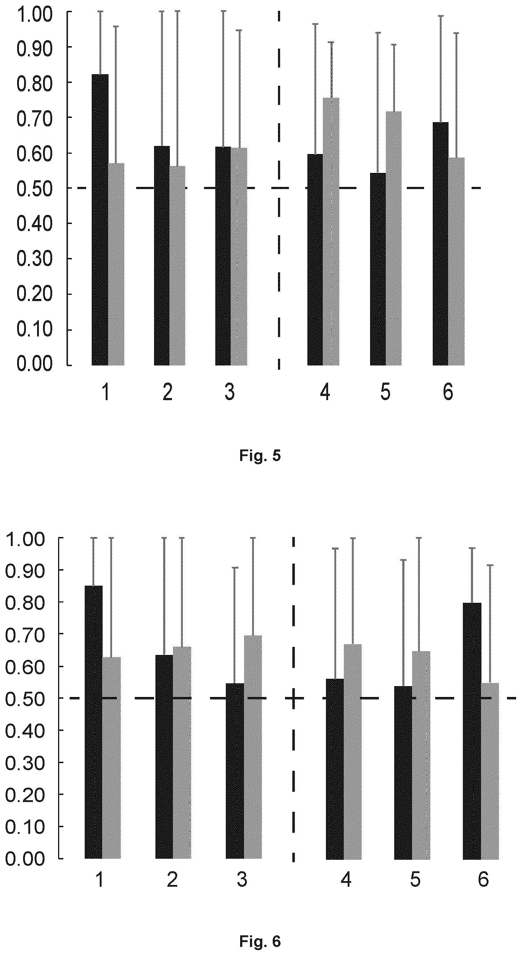

**Fig. 5. Tremor scores of electrical stimulation on wrist kinematics tremor amplitude for 4 ET patients right after stimulation.** Results from the post-ASSESS after the stimulation trials. (1-stimulated wrist, 2 ipsilateral elbow, 3 ipsilateral shoulder, 4-contralateral shoulder, 5 contralateral elbow, 6-contralateral wrist). 0.5 represents no change in tremor amplitude, 1 represents 100% tremor reduction and 0 represents 100% tremor aggravation. Black bars: intramuscular stimulation; grey bars: surface stimulation.

**Fig. 6. Prolonged tremor scores of electrical stimulation on wrist kinematics tremor amplitude for 4 ET patients.** Results from the assessment 24h after stimulation trials. (1-stimulated wrist, 2 ipsilateral elbow, 3 ipsilateral shoulder, 4-contralateral shoulder, 5 contralateral elbow, 6-contralateral wrist). 0.5 represents no change in tremor amplitude, 1 represents 100% tremor reduction and 0 represents 100% tremor aggravation. Black bars: intramuscular stimulation; grey bars: surface stimulation.

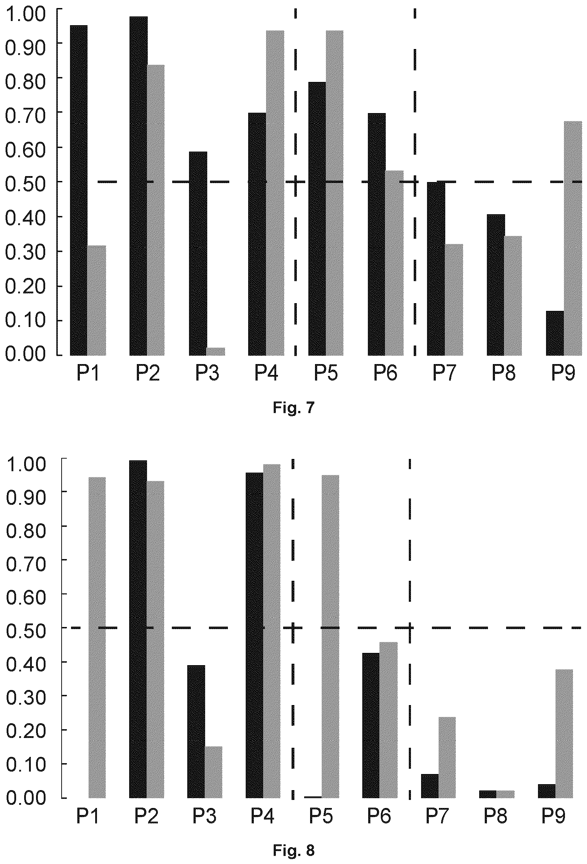

**Fig. 7. Effect of electrical stimulation for 9 ET patients (P1-P9) right after stimulation trials.** Tremor scores on wrist kinematics after the stimulation trials (post-ASSESS). 0.5 represents no change in tremor amplitude, 1 represents 100% tremor reduction and 0 represents 100% tremor aggravation. Black bars: intramuscular stimulation; grey bars: surface stimulation. P1-P4 received SATS and CON stimulation trials in the same session; P5-P6 received only SATS stimulation; P7-P9 received only CON stimulation.

**Fig. 8. Prolonged effect (24 hours) of electrical stimulation for 9 ET patients (P1-P9).** Tremor scores on wrist kinematics after the stimulation trials (post 24h-ASSESS). 0.5 represents no change in tremor amplitude, 1 represents 100% tremor reduction and 0 represents 100% tremor aggravation. Black bars: intramuscular stimulation; grey bars: surface stimulation. P1-P4 received SATS and CON stimulation trials in the same session; P5-P6 received only SATS stimulation; P7-P9 received only CON stimulation.

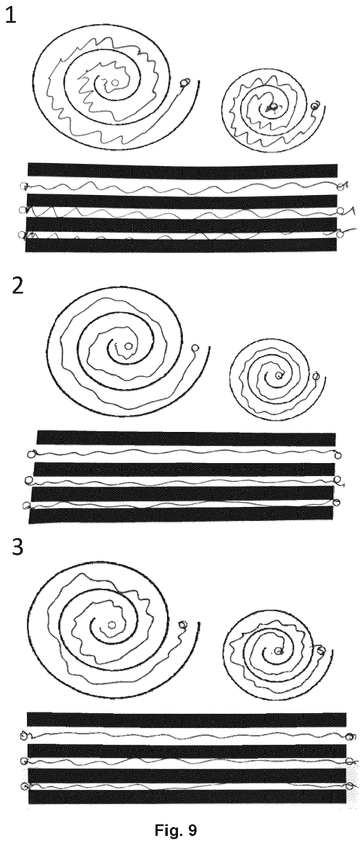

**Fig. 9. Spiral drawings from patient P6, who received SATS stimulation with intramuscular electrodes.** 1-Pre-assessment; 2-Post-Assessment; 3- post 24h-assessment.

## Examples

### Participants and Materials

### Participants

Eleven ET were recruited and clinically examined between April 2019 and January 2020. Inclusion criteria included diagnosis of ET with or without family history, according to Tremor Research Investigation Group, regardless of the disease duration and the current or previous treatment, presenting clinically objectionable postural tremor, age between 18-80 years, tremor affecting at least one of the upper limbs, with prominent wrist flexion-extension movement, absence of another neurological pathology that could produce abnormal movements or impaired mobility in the extremities, ability to understand the procedure; and sign the informed consent. Exclusion criteria included coexistence of other diseases that distort movement, mixed or complex tremors, with involvement of multiple muscles; concomitant important medical pathology; anticoagulant treatment.

### Materials

Each patient underwent two different sessions, at different weeks and in randomized order: 1) stimulation with thin-film multichannel intramuscular electrodes; and 2) surface stimulation.

For the intramuscular stimulation session (IntraStim), two intramuscular electrodes were acutely implanted in the muscle belly of flexor carpis radialis (FCR) and extensor carpis radialis (ECR) of the most affected side. The insertion of each intramuscular electrode was done with a hypodermic needle (25G, B. Braun AG, Germany) and guided by ultrasonography to guarantee its placement within the muscle belly.

For the surface stimulation session (SurfStim), round adhesive electrodes (3.2cm diameter, ValuTrode Cloth, Axelgaard Manufacturing, Denmark) were placed over the median and radial nerves of the most affected side through palpation, as previously described (Pascual-Valdunciel et al., Conf Proc IEEE Eng Med Biol Soc, Vol: 2019, Pages: 6267-6272). For both sessions, a surface ground squared electrode (5x5cm ,ValuTrode Cloth, Axelgaard Manufacturing, Denmark) was placed over the olecranon.

Stimulation strategies were controlled using a customized embedded processing unit including both an electromyography (EMG) amplifier and voltage electrical stimulator (OT Bioelettronica, Italy). Monopolar (intramuscular) or bipolar (surface) EMG signals were sampled at 1,000 Hz. Stimulation frequency was set at 100 Hz (Dideriksen et a/., Front. Neurosci., Apr. 2017, vol. 11: art. 178), with biphasic pulse width and maximum stimulation amplitude of 200 $\mu$s and 2.7 mA, and 400 $\mu$s and 5 mA, for intramuscular and surface electrodes, respectively (Muceli et al., J. Neural Eng., 2019, vol. 16, n. 2).

Additionally, bipolar surface electromyography (sEMG) electrodes were placed over the FCR and ECR after cleaning the skin with alcohol (Hermens et al., "European recommendations for surface electromyography: Results of the SENIAM Project," 1999). sEMG signals were acquired at 2,048 Hz with a biosignal amplifier (Quattrocento, OT Bioelettronica, Italy), which also recorded digital and synchronization signals from the stimulator unit and kinematics measurement system. Wet wristbands were secured around the wrist of the stimulated side and used as reference for all EMG recordings.

Kinematics of upper limbs joints were assessed through seven Inertial Measurements Units (IMUs) (Technaid S.L., Spain): two IMUs were placed over dorsal side of each hand, two over dorsal sides of each forearm, two were placed over the lateral side of each arm and one over the chest, which worked as reference. Raw quaternions data were sampled at 50 Hz and stored for offline analysis.

*Procedures*

For each different experimental session (each one on different weeks), IntraStim or SurfStim was delivered on each patient. The order of the sessions were randomized. At the beginning of each session, patients were instrumented with EMG and stimulation electrodes, as well as IMUs.

Subjects were asked to keep their arms pronated and outstretched, or with their elbows flexed and pronated facing both hands, for each trial. For each subject, the position that elicited higher tremor amplitude at wrist was selected. To assess basal tremor (pre-ASSESS trial), each participant was then asked to hold their upper limbs in the same posture that would elicited higher tremor amplitude, for 60 seconds, while IMUS and EMG data were recorded.

Then, stimulation parameters were calibrated. For IntraStim, a similar procedure to that described in Muceli et al., (J. Neural Eng., 2019, vol. 16, n. 2) was applied to find motor threshold (MT) and perception threshold (PT) of both ECR and FCR. The total current delivered by the three stimulating points never exceeded 2.7 mA, for safety reasons. In the case of SurfStim, MT and PT were also identified, and maximum current delivered over nerves was 5 mA maximum. For both types of stimulation, intensity delivered in stimulation trials was usually 0.1 mA below MT or the maximum current allowed for each type of stimulation (2.7 mA for intramuscular and 5 mA for surface stimulation), for those cases where MT was higher than maximum allowed stimulation.

After setting stimulation parameters, the session proceeded with several stimulation trials, each one lasting 60 seconds. For each of these trials, subjects were asked to keep the same posture as in pre-ASSESS. Stimulation trials started with 10 seconds of tremor recording, followed by two 30-seconds windows in which a different stimulation strategy was turned ON or OFF (randomly assigned order). During ON periods, a given stimulation condition was delivered to the patient, whereas no stimulation was applied during OFF periods. Each patient completed at least six stimulation trials and was blinded to the stimulation condition applied. In some cases, patients did not feel any sensation when stimulation was delivered.

The two different stimulation strategies applied in this study were selective and adaptive timely stimulation (SATS - the method of the invention) and continuous stimulation (CON):

- SATS was based on alternating EMG recording with electrical stimulation time windows (Fig. 1). It started with the analysis of 1s windows of FCR and ECR EMG data, demodulation of both signals to extract the main frequency of tremor, as a simplification of a previous method (Dideriksen et al., Front. Neurosci., Apr. 2017, vol. 11: art. 178). If signals' frequencies were in the range 3-12 Hz, tremor was detected and the stimulation was enabled for the next 2 seconds. During this window, RMS of 10 ms EMG epochs was computed for both FCR/ECR and, if any of the RMS values exceeded the adaptive threshold, then a short stimulation burst would be delivered to the antagonist muscle. This method considers that both antagonists can be in-phase and, therefore, be stimulated at the same time (Puttaraksa et al., J. Neurophysiol. 2019, vol. 122, no. 5, pp. 2043-2053,). After the stimulation burst, the short RMS recording windows were consecutively computed and stimulation would be delivered if tremorgenic activity was detected. Once the 2s stimulation window was past, 1s recording window was activated again to assess tremorgenic status and the same cycle repeated again.
- CON stimulation consisted of 1s recording EMG window followed by 2s simultaneous stimulation of both antagonists.

The first four patients enrolled for the study received SATS intermingled with CON stimulation, in randomized order, for both sessions. Two other patients underwent SATS while the other three underwent CON stimulation trials alone in each session, seeking to discriminate the effects of each stimulation condition. At the end of each session, the effect of stimulation on kinematics was assessed through IMUs (post-ASSESS), with patients holding their upper limbs as in pre-ASSESS. A final assessment (post24-ASSESS) was also performed 24h after each session to assess possible prolonged effects on tremor.

*Data Analysis*

Raw data from IMUs (quaternions) were converted into Euler angles and filtered in the tremor band [3-12 Hz], which allowed calculation of angle displacement for wrist, elbow and shoulder. Power Spectral Density (PSD) method (2s Hamming window and 50% segment overlap) was applied to quantify tremor amplitude in the assessments and stimulation trials. For the wrists, PSD for flexion-extension was assessed, whereas averaged PSD for the 2 and 3 degrees of freedom was computed for elbows and shoulders, respectively. PSD values were used to compute different tremor scores, depending on the pairs of conditions compared, according to the following equation:

$$Tremor\ score = 0.5 + 0.5 \times \frac{condition\ A - condition\ B}{max(condition\ A; condition\ B)}$$

(Equation I)

where condition A and B represent PSD of two conditions being compared. According to Equation I, a score equal to 1 would correspond to 100% tremor reduction, a score equal to 0.5 would correspond to no tremor change, and a score equal to 0 would correspond to 100% tremor aggravation. Equation I was applied to compare different conditions, such as the different assessments, ON/OFF periods, among others, through kinematics.

*Statistical analysis*

One-sample t-tests were computed to compare the effects of each type of stimulation on acute tremor reduction. Independent samples t-tests where applied to compare differences on acute tremor reduction scores between stimulation conditions (SATS IM vs SATS SF, SATS IM vs CON IM, SATS SF vs CON SF) for each joint. Statistical significance was set with $p$-value < 0.05.

**Example 1 - Acute effects**

Wrist flexion / extension angles during pre-ASSESS, during SATS (the method of the invention) stimulation trial and during post-ASSESS and pos24-ASSESS of a representative patient are depicted in Fig. 2. Acute tremor reduction for this specific subject can be observed in Fig. 2 (see block 2 compared to block 1).

Regarding all patients assessed in this study, acute tremor reduction scores (representing the ratio between tremor on the 30-second windows OFF and tremor on the 30-second windows ON across trials) for all joints are presented in Fig. 3. SATS IntraStim was the only condition achieving significant acute tremor reduction ($0.63\pm0.03$; $p$<0.01; equivalent to 26% tremor reduction) at the stimulated wrist, which was significantly higher compared to the other 3 conditions (SATS SurfStim, CON IntraStim and CON SurfStim; $p$<0.05 for the three comparisons). Another interesting observation was that tremor reduction score was significantly higher ($p$<0.01) for SATS SurfStim ($0.53\pm0.08$) than CON SurfStim strategy ($0.35\pm0.07$). In fact, CON strategy not only did not acutely reduce tremor amplitude at the stimulated wrist or any other joint, but it also aggravated wrist tremor during surface stimulation ($p$<0.05 for CON SurfStim session).

SATS IntraStim also achieved, on average, acute tremor reduction on the ipsilateral (stimulated) elbow ($0.60\pm0.10$) and shoulder ($0.58\pm0.08$) (Fig. 3-2,3), although these values were not significantly higher than 0.5 ($p$-value = 0.06). On the other hand, there was no significant acute tremor reduction for any of the contralateral (non-stimulated side) joints (Figs. 3-4,5,6).

Fig. 4 represents the ratio between tremor during OFF period of each trial and tremor during stimulation (2-second time windows when stimulation bursts were delivered during the 30-second ON period) of each trial. Also for this specific analysis, the highest acute tremor reduction was registered for the stimulated wrist with SATS IntraStim combination ($0.66\pm0.09$; $p$<0.05). None of the CON combinations achieved acute tremor reduction (Fig. 4).

**Example 2 - 24 hours effect**

Four patients (P01 - P04) underwent random trials of SATS and CON stimulation for both stimulation sessions (IntraStim and SurfStim) and post-ASSESS showed an effect on the stimulated wrist at the end of the session (Fig. 5 and Fig. 2). Despite the reduced sample size (n=4) and variability of tremor, mean tremor reduction in the stimulated wrist was higher after IntraStim sessions ($0.82\pm0.2$) compared to after SurfStim sessions ($0.57\pm0.39$). Tremor score was on average higher than 0.5 at the end of both sessions (post-ASSESS) and all assessed joints (Fig. 5).

Tremor reduction was still observed 24h after experimentation in 3 out of these 4 patients, with an average post24-ASSESS ipsilateral wrist tremor reduction score of $0.85\pm0.22$ for IntraStim session and $0.63\pm0.45$ for Surf Stimulation (Fig. 6 and representative example in Fig. 2).

In the other five patients, we applied just one simulation strategy (IntraStim or SurfStim) to discriminate the effect of each stimulation strategy on prolonged effects. There was tremor reduction at the stimulated wrist of the two patients that only received SATS during IntraStim session (average tremor score $0.76\pm0.07\%$ - Fig. 7), while the three patients receiving CON during IntraStim session showed tremor aggravation (average $0.35\pm0.2$ - Fig. 7).

Regarding the 24h effect, except for one of the 2 patients that received SATS SurfStim, all other patients aggravated tremor 24h after sessions. However, in general CON strategy alone aggravated tremor compared to SATS strategy 24h after sessions (Fig. 8). Spiral drawings also demonstrated improvements right after and 24h after SATS IntraStim (Fig. 9).

**Claims**

1. A method for a window-based control of a neuroprosthetic device comprising the steps of:

    a) obtaining a set of electromyography (EMG) signals from at least one pair of EMG electrodes;
    b) filtering the EMG signals obtained by using a low-pass filter;
    c) obtaining envelopes of the filtered EMG signals;
    d) determining two or more local maxima of the envelopes obtained;
    e) calculating period and frequency of tremorgenic bursts of each muscle based on said local maxima;
    f) determining an adaptive EMG threshold of tremor for each EMG signal, which is computed as root mean square (RMS) of the EMG signals obtained in step a) multiplied by a gain between 0.5 and 2;

    wherein when frequencies of tremorgenic bursts of one or more muscles are in the range of 3-12 Hz, is determined that tremor is present and a stimulation mode is enabled, which further comprises the steps of:

    i) setting a stimulation time-window, which has a duration inversely proportional to the period of tremorgenic burst, such that the shorter the tremorgenic burst, the longer the stimulation time-window;
    ii) obtaining one or more updated EMG signals of one or more muscles from the EMG electrodes;
    iii) obtaining an updated RMS value of a measuring time window of each updated EMG signal;
    iv) comparing each updated RMS value with the adaptive EMG threshold;
    v) determining the existence of tremor in an agonist muscle if the RMS value of an EMG electrode placed on said agonist muscle is above the adaptive EMG threshold,
    vi) generating an activation signal on the neuroprosthetic device placed in an antagonist muscle, with respect to the agonist muscle suffering a tremor, and repeating steps (ii) to (v) until the duration of the stimulation time-window is finished.

2. The method according to claim 1, wherein the low-pass filter is a Butterworth filter.

3. The method according to claims 1 and 2, further comprising a step of applying a Hilbert transformation to the envelope obtained in step c) for obtaining an improved envelope.

4. The method according to any of claims 1 to 3, wherein the step a) obtains the EMG signal during a period of 0.5 to 2 seconds.

5. The method according to any of claims 1 to 4, wherein the step ii) obtains the updated EMG signal during 3 to 15 milliseconds.

6. The method according to any of claims 1 to 5, wherein the step v) generates the activation signal during 5 to 20 milliseconds.

7. The method according to any of claims 1 to 6, wherein steps i) to vi) are performed during 30 seconds before repeating step a).

8. The method according to any of claims 1 to 7, further comprising a step of calculating the intensity and duration of the activation signal of the neuroprosthetic device based on the EMG signal and below the motor threshold.

9. A computer program adapted to perform the steps of the method of any of claims 1 to 8.

10. A computer readable storage medium comprising the computer program of claim 9.

EP 3 912 677 A1

Fig. 1

Fig. 2

Fig. 3

Fig. 4

Fig. 5

Fig. 6

Fig. 7

Fig. 8

Fig. 9

Europäisches
Patentamt

European
Patent Office

Office européen
des brevets

# EUROPEAN SEARCH REPORT

Application Number

EP 20 38 2416

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DOSEN STRAHINJA ET AL: "Online Tremor Suppression Using Electromyography and Low-Level Electrical Stimulation", IEEE TRANSACTIONS ON NEURAL SYSTEMS AND REHABILITATION ENGINEERING, IEEE SERVICE CENTER, NEW YORK, NY, US, vol. 23, no. 3, 1 May 2015 (2015-05-01), pages 385-395, XP011580859, ISSN: 1534-4320, DOI: 10.1109/TNSRE.2014.2328296 [retrieved on 2015-05-08] * page 386, left-hand column, lines 27-36 * * page 386, right-hand column, lines 10,49-54 * * page 387 * * figures 2-3 * | 1-10 | INV. A61N1/36 ADD. A61B5/0488 A61B5/04 |
| A | US 9 375 573 B2 (DILORENZO DANIEL JOHN [US]; CYBERONICS INC [US]) 28 June 2016 (2016-06-28) * column 13, lines 1-19 * | 1-10 | |
| A | WO 2020/006048 A1 (CALA HEALTH INC [US]) 2 January 2020 (2020-01-02) * paragraphs [0220] - [0223] * | 1-10 | TECHNICAL FIELDS SEARCHED (IPC) A61N A61B |

The present search report has been drawn up for all claims

| Place of search | Date of completion of the search | Examiner |
|---|---|---|
| Munich | 23 October 2020 | Rogala, Tomasz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 38 2416

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2020

| Patent document cited in search report | | Publication date | Patent family member(s) | Publication date |
|---|---|---|---|---|
| US 9375573 | B2 | 28-06-2016 | NONE | |
| WO 2020006048 | A1 | 02-01-2020 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060212089 A
- US 2006217781 A
- US 2010087698 A
- US 2011112394 A1
- US 2011184489 A
- US 2017014625 A
- US 20140336722 A


**Non-patent literature cited in the description**

- **SHANKER V.** *BMJ,* August 2019, 14485
- **FERREIRA et al.** *Mov Disord.,* 2019, vol. 34 (7), 950-958
- **PASCUAL-VALDUNCIEL et al.** *Conf Proc IEEE Eng Med Biol Soc,* 2019, 6267-6272
- **DIDERIKSEN.** *Front. Neurosci.,* April 2017, vol. 11
- **MUCELI et al.** *J. Neural Eng.,* 2019, vol. 16 (2
- **HERMENS et al.** *European recommendations for surface electromyography: Results of the SENIAM Project,* 1999
- **DIDERIKSEN et al.** *Front. Neurosci.,* April 2017, vol. 11
- **PUTTARAKSA et al.** *J. Neurophysiol.,* 2019, vol. 122 (5), 2043-2053